# EUROPEAN PATENT APPLICATION

(11) **EP 4 417 157 A1**
(43) Date of publication of application: **21.08.2024**
(21) Application number: 22913459.8
(22) Date of filing: 31.08.2022
(51) Int. Cl.: A61B 34/37

(54) **MAIN END OPERATING DEVICE AND SURGICAL ROBOT**

(30) Priority: 31.12.2021 CN 202111665845
(71) Applicant: Cornerstone Technology (Shenzhen) Limited, Shenzhen, Guandong 518066 (CN)
(72) Inventor: WANG, Zerui, Shenzhen, Guangdong 518066 (CN)
(74) Representative: Balder IP Law, S.L.
(86) International application number: PCT/CN2022/116385
(87) International publication number: WO 2023/124179

(57) **Abstract**

Disclosed in the present disclosure are a mater-end operating device and a surgical robot. The master-end operating device includes a device body, a pair of operating mechanisms, a movable member and an elastic member. The pair of operating mechanisms are pivotably connected to two opposite sides of the device body respectively, and a respective operating mechanism of the pair of operating mechanisms is provided with a respective driving part. The movable member is at least partially disposed within the device body and movable in an axial direction of the device body, where the movable member is provided with follower parts, each of which is configured to interfere with the respective driving part to enable the movable member to move in the axial direction under an action of the respective operating mechanism. The respective follower part has a first constraint surface, which includes at least one bent portion recessed toward a central plane of the device body. The elastic member is configured to provide an elastic force to drive the movable member to move toward a top end of the master-end operating device.

## Description

### CROSS REFERENCE TO RELATED APPLICATIONS

This application claims priority to Chinese Patent Application No. CN202111665845.9, entitled "MASTER-END OPERATING DEVICE AND SURGICAL ROBOT," filed on December 31, 2021, which is incorporated by reference herein in its entirety.

### TECHNICAL FIELD

The present disclosure relates to the technical field of medical devices, and in particular, to a master-end operating device and a surgical robot.

### BACKGROUND

Medical and surgical micro-instruments have the advantages of accurate positioning, stable operation, high dexterity, large working range, radiation and infection protection and the like, and are widely used in various surgeries. The use of surgical micro-instruments helps to improve the precision of the surgeon's operation, solves problems of trembling, fatigue, and musculo-neurological feedback of the surgeon's hand, and enables the surgeon to perform surgical operation in the most comfortable state, which is of great value for improving the success rate of surgery and alleviating the patient's pain. In recent years, the research of surgical micro-instruments has become a new field of medical device application.

The surgeon generally operates a master hand of a master robot on the surgeon's side, and the positions and postures of the surgeon's hand and the opening and closing of the surgeon's fingers are detected and mapped, at a certain magnification ratio, to motions of a slave hand of a slave robot on the patient's side, enabling the surgeon to operate the surgical instrument with dexterity and ease.

The end of the master hand is generally configured with a three-axis gimbal to measure the posture of the hand, and a grip handle is provided at the end of the gimbal, so that the surgeon controls an angle of opening and closing of an instrument hold by the slave hand by controlling the grip handle, to achieve operations of cutting, suturing, and the like. Therefore, the grip handle is a key component for high-frequency use in robotic surgery.

The tactile feedback provided by the grip handle to the operator is critical to the operator's operational judgment. In practice, for example, when operating a clamp, a master-hand grip handle generally simulates, by some means, the sensation of a two-stage force on slave-hand clamping jaws. That is, a first stage force is existed from a position where the clamping jaws are at a maximum opening angle to a position where the clamping jaws move back to a state of nearly tight clamping, achieving the mapping control of positions of opening and closing of the slave-hand clamping jaws. Generally, at the end of a first-stage travel of the master-hand grip handle, the slave-hand clamping jaws with the mapped motions has already, or nearly, reached to a closing state, but has a relatively low output force. A second stage force is existed from a position where the clamping jaw has a slight angle in a state of nearly tight clamping to a position where the clamping jaw is in a state of full clamping, tight clamping or forceful cutting, providing the operator with the tactile sensation of tight clamping. Typically, an angle for switching between the two stage forces of the master-hand grip handle occurs when an included angle between two clamping jaws of the grip handle is within 10°, thus indicating and providing feedback to the surgeon that a tight clamping or cutting operation is being performed with a considerable output force. The angle for switching and the strength of the feedbacked the two-stage clamping forces are of great importance for physician operation, and are key points in control of the master-hand of a medical robot.

### SUMMARY

A series of concepts in simplified form are introduced in the section "SUMMARY," and will be described in further detail in the section "DETAILED DESCRIPTION OF THE EMBODIMENTS". The section "SUMMARY" of the present disclosure is not intended to limit the key features and essential technical features of the technical solutions sought to be protected, let alone to being intended to determine the protection scope of the technical solutions sought to be protected.

Embodiments of a first aspect of the present disclosure provide a master-end operating device for a surgical robot. The master-end operating device includes a device body; a pair of operating mechanisms, pivotably connected to two opposite sides of the device body respectively, a respective operating mechanism of the pair of operating mechanisms being provided with a respective driving part; a movable member at least partially disposed within the device body and movable in an axial direction of the device body; where the movable member is provided with follower parts, a respective follower part of the follower parts is configured to interfere with the respective driving part to enable the movable member to move in the axial direction under the action of the respective operating mechanism, the respective follower part has a first constraint surface, and the first constraint surface includes at least one bent portion along a starting end to a terminal end of the first constraint surface, the at least one bent portion being recessed toward a central plane of the device body; an elastic member, configured to provide an elastic force to drive the movable member to move toward a top end of the master-end operating device.

In the master-end operating device according to the embodiments of the first aspect of the present disclosure, the structure is simple, the strength of the connecting members is high, the transition of the force can be accurately controlled, the output of the clamping force can be easily controlled, and the symmetrical movement of the pair of operating mechanisms can be easily maintained.

In some embodiments, the first constraint surface includes a first constraint segment from the starting end to the at least one bent portion, and a second constraint segment from the at least one bent portion to the terminal end, and a slope at a junction between the first constraint segment and the at least one bent portion is smaller than a slope at a junction between the at least one bent portion and the second constraint segment with respect to a radial cross section of the device body.

In some embodiments, at least one of the first constraint segment and the second constraint segment is a flat or curved surface.

In some embodiments, the respective follower part further has a second constraint surface spaced from the first constraint surface, a space for accommodation and movement of the respective driving part is defined between the second constraint surface and the first constraint surface, and an included angle is formed between the second constraint surface and the radial cross section.

In some embodiments, the second constraint surface includes a third constraint segment and a fourth constraint segment connected to the third constraint segment, the third constraint segment opposite the first constraint segment, the fourth constraint segment opposite the second constraint segment, and the second constraint surface being disposed parallel to the first constraint surface.

In some embodiments, the respective driving part includes a driving shaft disposed between the first constraint surface and the second constraint surface.

In some embodiments, the respective operating mechanism includes a body, each of two ends of the driving shaft is provided with a bearing. An outer ring of the bearing is connected to the body, and an inner ring of the bearing is connected to the driving shaft.

In some embodiments, the movable member includes: a movable block, disposed in the device body, the follower parts being disposed on the movable block, and an outer periphery of the movable block being restricted by an inner periphery of the device body; and a movable rod, extending out of the device body in a direction away from the top end from the movable block.

In some embodiments, a through hole is defined at a top of the device body; the movable rod penetrates the movable block, extends to the top end, and extends into the through hole; and the master-end operating device further includes a positioning sleeve extending into the through hole and sleeved on the movable rod, an outer periphery of the positioning sleeve being restricted by an inner periphery of the through hole.

In some embodiments, the elastic member is configured as a compression spring; and the master-end operating device includes a mounting base, the mounting base is disposed in the device body, and is farther away from the top end of the master-end operating device than the movable block, and the elastic member is connected between the movable block and the mounting base, or, the elastic member is connected between the pair of operating mechanisms and the device body.

In some embodiments, the pair of operating mechanisms is symmetrical about a central plane of the master-end operating device; and/or the respective operating mechanism includes a body and a finger cuff disposed on the body.

Embodiments of a second aspect of the present disclosure provide a surgical robot. The surgical robot includes a slave-end clamp, the master-end operating device described in the first aspect, the respective follower part of the master-end operating device including the first constraint surface having a first constraint segment and a second constraint segment; and a control device, which is signally connected to the master-end operating device and the slave-end clamp and configured to: in response to the respective driving part interfering with the first constraint segment and moving in a direction toward the second constraint segment, control the slave-end clamp to gradually transition from an open state to a clamping state; in response to the respective driving part being located at a joint of the first constraint segment and the second constraint segment, control the slave-end clamp to transition into the clamping state; and in response to the respective driving part interfering with the second constraint segment and moving in a direction away from the first constraint segment, control a clamping force of the slave-end clamp to gradually increase.

The surgical robot according to the present disclosure has the same technical effects as those of the master-end operating device as described in the first aspect.

### BRIEF DESCRIPTION OF THE DRAWINGS

The following accompanying drawings of the present disclosure are hereby used as part of the present disclosure for understanding the invention. The accompanying drawings show embodiments of the present disclosure and descriptions thereof, which are used to explain the principles of the present disclosure.
FIG. 1 is a schematic diagram illustrating a front view of a master-end operating device according to an embodiment of the present disclosure.
FIG. 2 is a partial perspective view of the master-end operating device in FIG. 1.
FIG. 3 is a schematic diagram illustrating a side view of the master-end operating device in FIG. 1.
FIG. 4 is a schematic diagram illustrating a sectional view taken along line A-A' of the master-end operating device in FIG. 1.
FIG. 5 is a schematic diagram illustrating an enlarged view of part C in FIG. 4.
FIG. 6 is a schematic diagram illustrating a sectional view taken line B-B' of the master-end operating device in FIG. 3.

### DETAILED DESCRIPTION OF THE EMBODIMENTS

In the following description, many specific details are given to provide a more thorough understanding of the present disclosure. However, it would be apparent to those skilled in the art that the present disclosure may be implemented without one or more of these details. In other examples, some technical features that are well known in the art are not described to avoid confusion with the present disclosure.

For a thorough understanding of the present disclosure, the following provides a detailed description. It should be understood that these embodiments are provided to make the disclosure of the invention thorough and complete, and to adequately convey the idea of these exemplary embodiments to those skilled in the art. Apparently, the embodiments of the present disclosure are not limited in their implementation to particular details familiar to those skilled in the art. Preferred embodiments of the present disclosure are described in detail below. However, the present disclosure may also have other embodiments in addition to these detailed descriptions.

It should be noted that the terms used herein are intended only to describe specific embodiments and are not intended to limit the exemplary embodiments according to the present disclosure. As used herein, the singular form is also intended to include the plural form, unless the context clearly indicates otherwise. Furthermore, it should be further understood that the terms "includes," "including," "comprises," and/or "comprising," when used herein, specify the presence of stated features, integers, steps, operations, elements, and/or components, but do not preclude the presence or addition of one or more other features, integers, steps, operations, elements, components, and/or groups thereof.

Ordinal numbers such as "first" and "second" quoted in the present disclosure are merely identifications, and do not have any other meanings, such as a specific order. Also, for example, the term "first element" does not by itself imply the presence of "second element," nor does the term "second element" by itself imply the presence of "first element." The terms "upper," "lower," "front," "rear," "left," "right," "inner," "outer" and similar expressions used herein are for the purpose of illustration and not limitation.

At present, clamping jaws with a two-stage force mostly simulate the two-stage force by the principle of springs with different stiffness and length, and motions of two clamping jaws are coupled together by a crank connecting rod, paired meshing gears and other mechanisms to simulate symmetrical clamping. However, it is found that there are many problems with this kind of clamping jaws. For example, in a compact space, it is difficult to accurately match placement positions and compression distances of two springs with different stiffness; an angle measurement component is complex in structure and occupies a large space; a connecting rod has low strength and is subjected to an asynchronous stress; and gears are prone to meshing clearance, which leads to inaccuracy and the like. To overcome or improve at least one of the foregoing problems, the present disclosure provides a master-end operating device and a surgical robot, optionally implemented by one or more of the following embodiments.

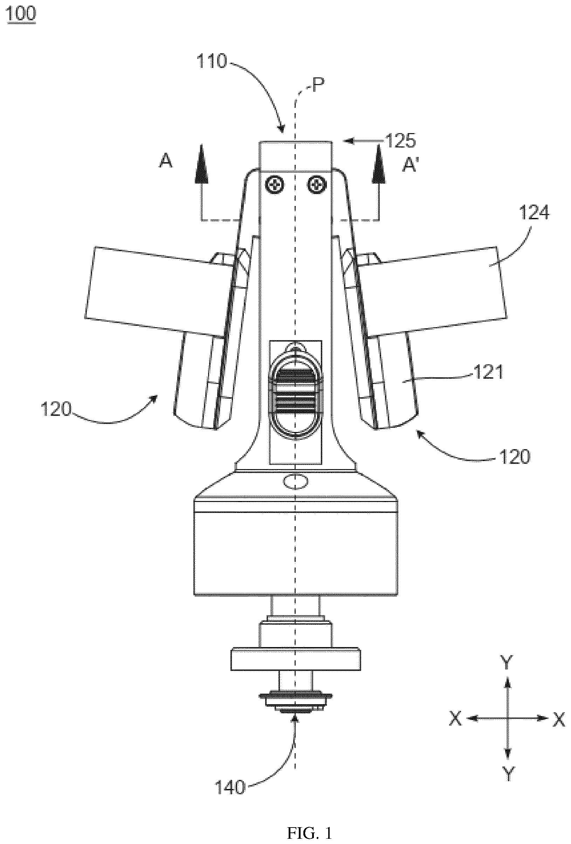

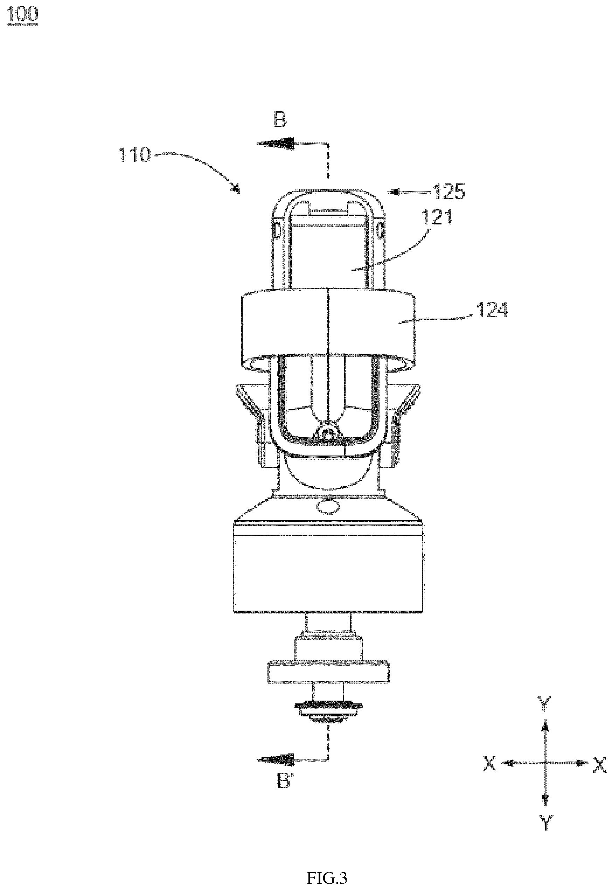

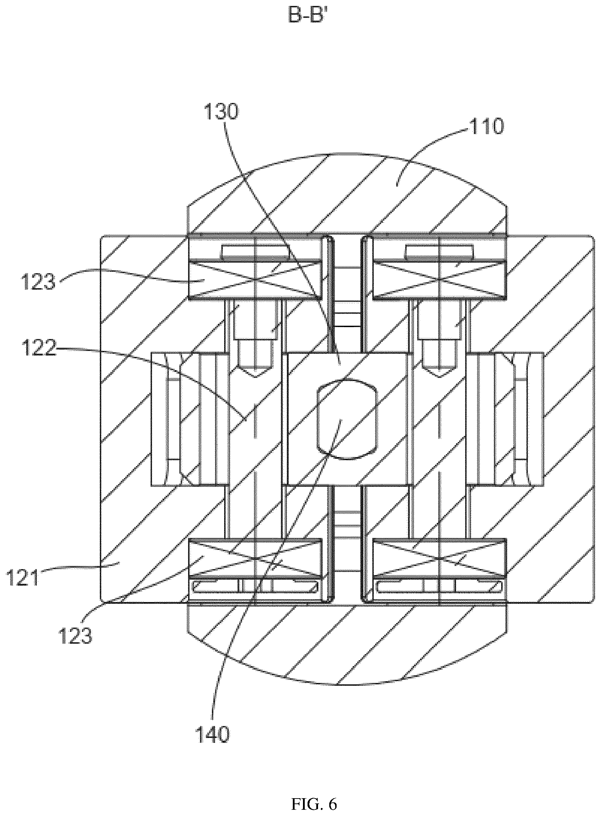

Exemplary embodiments according to the present disclosure are described in more detail with reference to FIGS. 1 to 6.

Referring to FIGS. 1 to 4, a master-end operating device 100 of an optional embodiment of a first aspect of the present disclosure includes a device body 110, a pair of operating mechanisms 120, a movable member and an elastic member 150.

The pair of operating mechanisms 120 is pivotably connected to the device body 110, and is positioned on two opposite sides of the device body 110 respectively. For example, a respective operating mechanism 120 of the pair of operating mechanisms 120 may be pivotably connected to the device body 110 through a pivot shaft 112.

The respective operating mechanism 120 is provided with a driving part. In some embodiments, the respective operating mechanism 120 includes a body 121 and a finger cuff 124 disposed on the body 121 in order to facilitate operation and avoid easy release grip of the operating mechanism 120. Exemplarily, the finger cuff 124 may be customized according to shapes of different fingers to facilitate finger insertion operations. In an optional implementation, the pair of operating mechanisms 120 is disposed symmetrically about a central plane P of the master-end operating device 100.

The movable member is at least partially disposed within the device body 110, and is configured to be movable in the axial direction of the device body 110. In an optional implementation, a mounting base 160 is provided within the device body 110, and is farther away from a top end 125 of the master-end operating device 100 than the movable block 130. The elastic member 150 is connected between the movable member and the mounting base 160, and is configured as a compression spring to provide an elastic force that moves the movable member upward or toward the top end 125 of the master-end operating device 100.

On the premise of a small included angle between the respective operating mechanism 120 and the device body 110, a compression amount of the elastic member 150 can be set to be small, so that an output force of the spring is close to a constant value, thereby avoiding the introduction of a variation factor of the spring force to make the output force more stable.

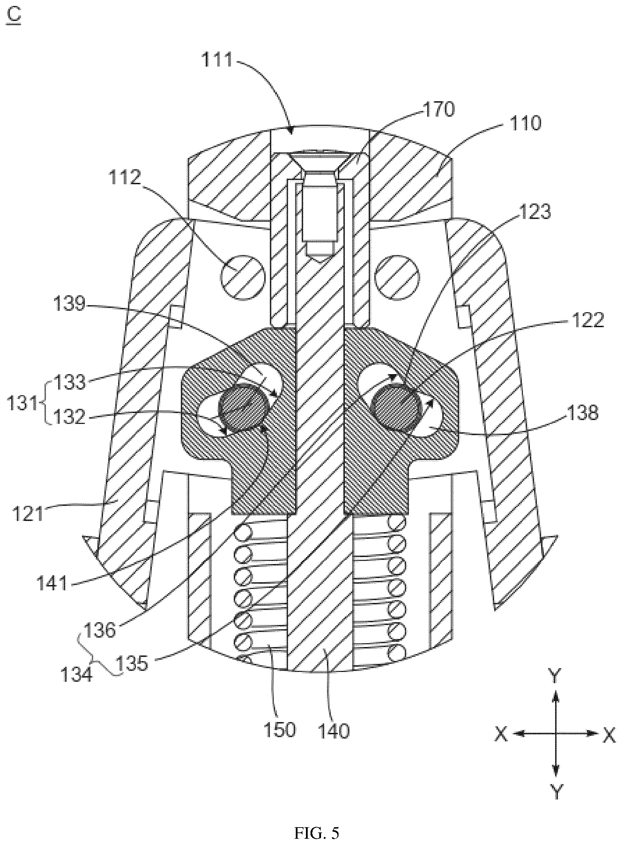

The movable member is provided with a follower part capable of interfering with the driving part. Therefore, the follower part enables the movable member to move in the axial direction under the action of the driving part. In some embodiments, a pair of follower parts is also symmetrically arranged corresponding to the pair of operating mechanisms 120. A single follower part has a first constraint surface 131 having at least one bent portion 141 (shown in FIG. 5). The bent portion 141 is recessed toward the central plane P of the device body 110. The central plane P refers to a plane that bisects the device body 110 and makes the pair of operating mechanisms 120 symmetrical.

Specifically, the first constraint surface 131 includes one bent portion 141. Thus, the first constraint surface 131 is divided into a first constraint segment 132 and a second constraint segment 133 by the bent portion 141. In other words, a portion of the first constraint surface 131 from a starting end to the bent portion 141 is configured as the first constraint segment 132 described above. A portion of the first constraint surface 131 from the bent portion 141 to a terminal end is configured as the second constraint segment 133. The starting end refers to an end of the follower part away from the central plane P, and the terminal end refers to an end of the follower part proximate to the central plane P.

Thus, the bent portion 141 is configured as a corner or a bent angle of the first constraint surface 131. Specifically, the bent portion 141 is a small segment of curved surface of the first constraint surface 131.

In some embodiments, an included angle between the first constraint segment 132 and a radial cross section of the device body 110 is smaller than an included angle between the second constraint segment 133 and the radial cross section. In other words, a slope at a junction between the first constraint segment 132 and the bent portion 141 is smaller than a slope at a junction between the bent portion 141 and the second constraint segment 133 with respect to the radial cross section of the device body 110.

The junction between the first constraint segment 132 and the bent portion 141 refers to a point at a joint of the first constraint segment 132 and the bend section 141, and the junction between the bent portion 141 and the second constraint segment 133 refers to a point at a joint of the bend section 141 and the second constraint segment 133.

When the bent portion 141 is a segment of curved surface, the two junctions are two ends of the bent portion 141 respectively. When the bent portion 141 is a junction point of the bent angle, the above two junctions are at the apex of the bent angle.

In some embodiments, the first constraint segment 132 and/or the second constraint segment 133 are flat or curved surfaces. In some embodiments, the first constraint segment 132 and/or the second constraint segment 133 are curved surfaces, and each of the first constraint segment 132 and the second constraint segment 133 has a curvature less than a curvature of the bent portion 141. Exemplarily, the first constraint segment 132 and/or the second constraint segment 133 are curved surfaces with a curvature approaching zero.

The interfering motion of the driving part with the first constraint segment 132 is mapped to a state transition process of the slave-end clamp from unclamping to clamping, i.e., approaching toward clamping (but not tight clamping) from an open state. The interfering motion of the driving part with the second constraint segment 133 is mapped to a state transition process of the slave-end clamp from clamping to tight clamping.

Thus, compared with the first constraint segment 132, the second constraint segment 133 has a large included angle that makes a component of a force applied by the operating mechanism 120 to the movable member in a Y direction (where the Y direction is a direction coplanar with the central plane P and perpendicular to an X direction) smaller and that makes an amount by which the movable member moves in the Y direction in response to an angle of rotation of the operating mechanism 120 smaller. In this way, the movable member can be moved by a relatively small distance through applying, by the operator, a greater force to the operating mechanism 120 to rotate the operating mechanism 120 by a greater angle, thereby facilitating improving the accuracy of the operation with respect to the clamping force of the slave-end clamp. In addition, there is an abrupt change in angle from the end of the first constraint segment 132 to the beginning of the second constraint segment 133, and the pressure of the spring suddenly increases, causing a sudden increase in the operation force felt by the user, so that the moving force at the slave-end clamping jaw or scissors also suddenly increases, thereby successfully completing the clamping or cutting action. In the whole process, the operator would have a two-stage force feeling which is very close to the real feeling of the hand using the clamping jaw or scissors.

In addition, when the movable member moves toward the top end 125 of the master-end operating device 100 under the action of the elastic force after release grip of the operator, the follower part is able to exert an action on the driving part so that the operating mechanism 120 can be reset with the driving part.

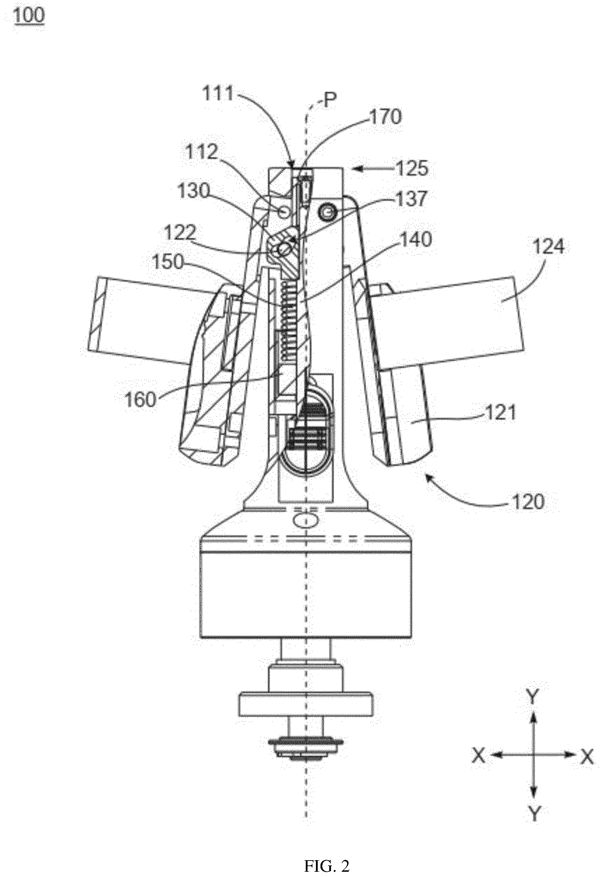

Specifically, referring to FIGS. 2, 4 and 5, the follower part may optionally further have a second constraint surface 134. The second constraint surface 134 is spaced from the first constraint surface 131 to form, between the second constraint surface 134 and the first constraint surface 131, a space for accommodating movement of the driving part, or forming a movement groove 137. The second constraint surface 134 forms an included angle with the radial cross section. Thus, the driving part can be restricted between the first constraint surface 131 and the second constraint surface 134.

Further optionally, the second constraint surface 134 includes a third constraint segment 135 and a fourth constraint segment 136 connected to the constraint segment 135. The third constraint segment 135 corresponds to the first constraint segment 132, the fourth constraint segment 136 corresponds to the second constraint segment 133, and an included angle between the third constraint segment 135 and the radial cross section of the device body 110 is smaller than an included angle between the fourth constraint segment 136 and the radial cross section.

In some embodiments, the first constraint surface 131 and the second constraint surface 134 are arranged in parallel. That is, the first constraint segment 132 is parallel to the third constraint segment 135, and the second constraint segment 133 is parallel to the fourth constraint segment 136.

In the case of having the second constraint surface 134, the elastic member 150 may alternatively be connected between the device body 110 and the operating mechanism 120 to provide an elastic force that allows the operating mechanism 120 to be reset. Thus, the driving part can interact with the second constraint surface 134 upon the release grip of the operator, thereby driving the movable member to move in a direction toward the top end 125 of the device body 110.

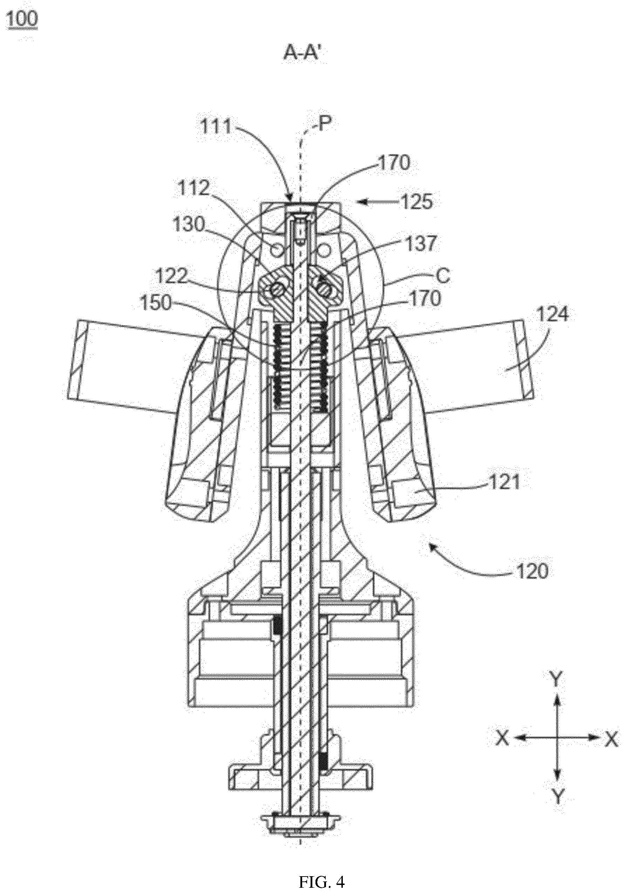

Referring further to FIGS. 4 and 5, the driving part includes a driving shaft 122 disposed between the first constraint surface 131 and the second constraint surface 134. Each of two ends of the driving shaft 122 is provided with a bearing 123, for example, a deep groove ball bearing. An outer ring of the bearing 123 is connected to the body 121, and an inner ring of the bearing 123 is connected to the driving shaft 122. In this way, when the driving shaft 122 of the driving part moves along the first constraint surface 131, contact friction can be reduced, and even sliding friction can be completely changed into rolling friction, thereby facilitating smooth operation.

A portion of the movement groove 137 between the first constraint segment 132 and the third constraint segment 135 forms a first movement segment 138, and a portion of the movement groove 137 between the second constraint segment 133 and the fourth constraint segment 136 forms a second movement segment 139. When the driving shaft 122 is located at the first movement segment 138, the slave-end clamp is in a clamping state, or, in a state transition process from unclamping to clamping. When the driving shaft 122 is located at the second movement segment 139, the slave-end clamp is in a tight clamping state, or, in a state transition process from clamping to tight clamping.

A distance between the first constraint surface 131 and the second constraint surface 134 is In some embodiments compatible with a diameter of the driving shaft 122. For example, the distance between the first constraint surface 131 and the second constraint surface 134 may be equal to, or slightly greater than, the diameter of the driving shaft 122. Thus, the driving shaft122 can be better restricted to move within the first movement segment 138 and the second movement segment 139.

The movable member includes a movable block 130 and a movable rod 140. The movable block 130 is disposed in the device body 110, and has an outer circumference restricted by the inner circumference of the device body 110, so that the movable block 130 can only move in the axial direction. The follower part is provided on the movable block 130. In the illustrated implementation, the follower part is configured as a groove provided on the movable block 130, such as a splayed groove which is symmetrically arranged.

The movable rod penetrates the movable block 130, a top end of the movable rod extends to the top of the device body 110, and a bottom end of the movable rod extends out of the device body 110 to be configured for connection with a slave-end clamp or a control device. The top of the device body 110 is provided with a through hole 111, and the movable rod extends into the through hole 111. The top of the device body 110 is further provided with a positioning sleeve 170 which extends into the through hole 111 and is sleeved on the movable rod 140. An outer circumference of the positioning sleeve 170 is restricted by an inner circumference of the through hole 111, so that the position of the movable rod 140 is also restricted, improving the stability of the movable restricted 140 in the Y direction, and the movable rod 140 can move up and down stably. Moreover, such configuration also improves the strength of the movable part.

According to the master-end operating device 100 of the present disclosure, the structure is simple, the strength of the connecting members is high, the transition of the force can be accurately controlled, the output of the clamping force can be easily controlled, and the symmetrical movement of the pair of operating mechanisms 120 can be easily maintained.

Embodiments of a second aspect of the present disclosure provide a surgical robot. The surgical robot includes a slave-end clamp, a master-end operating device 100 and a control device. The control device is signally connected to the master-end operating device 100 and the slave-end clamp.

The control device is configured to control the slave-end clamp to gradually transition from an open state to a clamping state when the driving part interferes with the first constraint segment 132 and moves in a direction toward the second constraint segment 133, to transition into the clamping state when the driving part is located at a joint of the first constraint segment 132 and the second constraint segment 133, and control a clamping force of the slave-end clamp to gradually increase when the driving part interferes with the second constraint segment 133 and moves in a direction away from the first constraint segment 132.

Unless otherwise defined, technical and scientific terms used herein have the same meanings as those commonly understood by those skilled in the art. The terms used herein are only used for describing specific embodiments and not intended to limit the present disclosure. Features described herein in one embodiment may be applied to another embodiment alone or in combination with other features, unless the features are not applicable in the another embodiment or otherwise noted.

The present disclosure has been described by way of the above-described embodiments, but it should be understood that the above-described embodiments are only for the purpose of illustration and description and are not intended to limit the present disclosure to the scope of the described embodiments. In addition, it should be understood by those skilled in the art that the present disclosure is not limited to the above-described embodiments, and that more variations and modifications may be made in accordance with the teachings of the present disclosure, all of these variations and modifications falling within the protection scope of the present disclosure. The protection scope of the present disclosure is defined by the appended claims and their equivalent scope.

## Claims

1. A master-end operating device for a surgical robot, comprising:
a device body;
a pair of operating mechanisms, pivotably connected to two opposite sides of the device body respectively, wherein a respective operating mechanism of the pair of operating mechanisms is provided with a respective driving part;
a movable member, at least partially disposed within the device body and movable in an axial direction of the device body, wherein the movable member is provided with follower parts, each respective follower part of the follower parts is configured to interfere with the respective driving part to enable the movable member to move in the axial direction under an action of the respective operating mechanism, the respective follower part has a first constraint surface, and the first constraint surface includes at least one bent portion along a starting end to a terminal end of the first constraint surface, the at least one bent portion being recessed toward a central plane of the device body; and
an elastic member, configured to provide an elastic force to drive the movable member toward a top end of the master-end operating device.

2. The master-end operating device according to claim 1, wherein the first constraint surface includes a first constraint segment from the starting end to the at least one bent portion, and a second constraint segment from the at least one bent portion to the terminal end, and a slope at a junction between the first constraint segment and the at least one bent portion is smaller than a slope at a junction between the at least one bent portion and the second constraint segment with respect to a radial cross section of the device body.

3. The master-end operating device according to claim 2, wherein at least one of the first constraint segment and the second constraint segment is a flat or curved surface.

4. The master-end operating device according to claim 2 or claim 3, wherein the respective follower part further has a second constraint surface spaced from the first constraint surface, a space for accommodation and movement of the respective driving part is defined between the second constraint surface and the first constraint surface, and an included angle is formed between the second constraint surface and the radial cross section.

5. The master-end operating device of claim 4, wherein the second constraint surface includes a third constraint segment and a fourth constraint segment connected to the third constraint segment, the third constraint segment opposite the first constraint segment, the fourth constraint segment opposite the second constraint segment, and the second constraint surface being disposed parallel to the first constraint surface.

6. The master-end operating device according to claim 5, wherein the respective driving part includes a driving shaft disposed between the first constraint surface and the second constraint surface.

7. The master-end operating device according to claim 6, wherein the respective operating mechanism includes a body, each of two ends of the driving shaft is provided with a bearing, an outer ring of the bearing is connected to the body, and an inner ring of the bearing is connected to the driving shaft.

8. The master-end operating device according to any one of claims 1 to 7, wherein the movable member includes:
a movable block, disposed in the device body, the follower parts being disposed on the movable block, and an outer periphery of the movable block being restricted by an inner periphery of the device body; and
a movable rod, extending out of the device body in a direction away from the top end from the movable block.

9. The master-end operating device according to claim 8, wherein:
a through hole is defined at a top of the device body;
the movable rod penetrates the movable block, extends to the top end, and extends into the through hole; and
the master-end operating device further includes a positioning sleeve extending into the through hole and sleeved on the movable rod, an outer periphery of the positioning sleeve being restricted by an inner periphery of the through hole.

10. The master-end operating device according to claims 8 or claim 9, wherein the elastic member is configured as a compression spring; and
wherein the master-end operating device includes a mounting base, the mounting base is disposed in the device body, and is farther away from the top end of the master-end operating device than the movable block, and the elastic member is connected between the movable block and the mounting base, or
wherein the elastic member is connected between the pair of operating mechanisms and the device body.

11. The master-end operating device according to claim 7, wherein at least one of:
the pair of operating mechanisms is symmetrical about a central plane of the master-end operating device; and
the respective operating mechanism further includes a finger cuff disposed on the body.

12. A surgical robot, comprising:
a slave-end clamp;
the master-end operating device according to any one of claims 1 to 11, wherein the respective follower part of the master-end operating device includes the first constraint surface having a first constraint segment and a second constraint segment; and
a control device, signally connected to the master-end operating device and the slave-end clamp and configured to:
in response to the respective driving part interfering with the first constraint segment and moving in a direction toward the second constraint segment, control the slave-end clamp to gradually transition from an open state to a clamping state;
in response to the respective driving part being located at a joint of the first constraint segment and the second constraint segment, control the slave-end clamp to transition into the clamping state; and
in response to the respective driving part interfering with the second constraint segment and moving in a direction away from the first constraint segment, control a clamping force of the slave-end clamp to gradually increase.
